# EUROPEAN PATENT APPLICATION

(11) **EP 1 743 762 A1**
(43) Date of publication of application: **17.01.2007**
(21) Application number: 05076601.3
(22) Date of filing: 13.07.2005
(51) Int. Cl.: B32B 3/20, B32B 5/02, B32B 27/32, A61F 13/15

(54) **Process and apparatus for producing breathable laminates**

(71) Applicant: Exten S.A., 6850 Mendrisio (CH)
(72) Inventor: Carlini, Luigi, 6924 Soregno (CH)
(74) Representative: Pizzoli, Antonio

(57) **Abstract**

Process and apparatus for producing breathable laminates (3), wherein a laminate (3) comprising a polymeric film (5) laminated with a nonwoven (6) is embossed by means of at least one embossing roller (8) provided with a plurality of points (9; 13) protruding from its cylindrical surface. The present invention also relates to laminates obtained by means of said process and/or apparatus.

## Description

The present invention relates to a process and an apparatus for producing breathable laminates, i.e. waterproof and permeable to water vapor, which can be used for manufacturing clothes or hygienic items, such as for example diapers, sanitary towels, etc. The present invention also relates to laminates obtained by means of said process and/or apparatus.

US 5865926 discloses a process and an apparatus for producing breathable laminates, wherein a laminate comprising a polymeric film laminated with a nonwoven is stretched by a stretcher after the lamination. The polymeric film comprises mineral particles, so that this stretching creates in the film microscopic pores which make the laminate breathable. Said stretcher comprises a pair of rollers, the cylindrical surfaces of which are provided with rectilinear grooves and ribs, mutually complementary, which are arranged in a parallel, diagonal or transversal manner with respect to the transport direction of the laminate. After the treatment with the stretcher, the surface of the laminate is lengthened and/or widened, with consequent problems for adapting the production line for the new dimensions of the laminate.

It is therefore an object of the present invention to provide a process and an apparatus which are free from said disadvantage. Said object is achieved with a process, an apparatus and a laminate, the main features of which are disclosed in claims 1, 11 and 28, respectively, while other features are disclosed in the remaining claims.

Thanks to the particular embossing, the process and the apparatus according to the present invention allow to improve the physical features of the laminate without modifying its external sizes. In particular, said process and apparatus can induce or increase the breathability of the laminate, especially if the latter comprises a polymeric film which includes fine mineral particles. As a matter of fact, when this particular laminate is embossed, its film is stretched causing microscopic pores close to the mineral particles, so as to make breathable a laminate which is not breathable or not very breathable.

The laminate is preferably embossed with a roller provided with points having particular shape, sizes and/or positions which allow to further improve its breathability without compromising its impermeability. Said embossing is carried out with particular temperatures, pressures and/or feeding speeds according to the material and/or the possible uses of the laminate, so as to optimize the features and/or the production of the latter.

Said embossing roller preferably presses the laminate against a particular elastomeric cylinder for avoiding breakings of its film. For this purpose, this film is preferably turned toward the elastomeric cylinder during the embossing, while the nonwoven is turned toward the embossing roller.

Further advantages and features of the process, apparatus and laminate according to the present invention will become clear to those skilled in the art from the following detailed and non-limiting description of some embodiments thereof with reference to the attached drawings, wherein:
- figure 1 shows a schematic view of a first embodiment of the apparatus;
- figure 2 shows an enlarged and partial front view of the surface of the embossing roller of the apparatus of figure 1;
- figure 3 shows an enlarged cross-sectioned view of plane III-III of figure 2;
- figure 4 shows an enlarged and partial front view of the surface of the embossing roller of a second embodiment of the apparatus; and
- figure 5 shows an enlarged cross-sectioned view of plane V-V of figure 4.

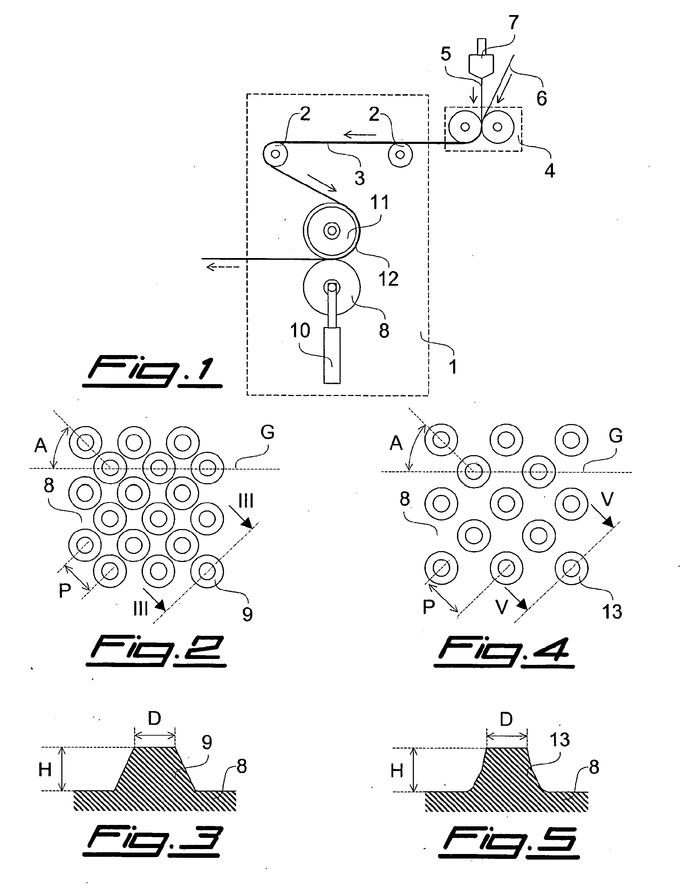

Referring to figure 1, it is seen that the apparatus 1 according to the invention comprises in a known way transport means, in particular a plurality of rollers 2, suitable for pulling a laminate 3 coming from a lamination device 4 in which at least one polymeric film 5 is laminated at least with a nonwoven 6. Film 5 comes in particular from an extruder 7.

With reference also to figures 2 and 3, it is seen that, according to the invention, apparatus 1 suitably comprises at least one embossing roller 8 which embosses laminate 3 by means of a plurality of points 9 which protrude from it cylindrical surface, so as to emboss the surface of laminate 3 with a dot pattern. The lamination and the embossing of laminate 3 are therefore carried out in a continuous manner one after the other.

Preferably, points 9 have a substantially frustoconical shape and are arranged along a plurality of lines and columns inclined with respect to a generatrix G of the cylindrical surface of roller 8, i.e. with respect to a transversal axis of laminate 3, with an angle A comprised between 40° and 50°, in particular 45°. Preferably, height H of points 9 is comprised between 0,7 and 0,9 mm, in particular 0,8 mm, while diameter D of their upper surface, having a substantially circular shape, is comprised between 0,6 and 0,8 mm, in particular 0,7 mm. Furthermore, pitch P between one point 9 and the four adjacent points is comprised between 1,3 and 1,6 mm, in particular 1,46 mm, while the area occupied by the upper surface of points 9 is a fraction comprised between 16% and 20%, in particular 18%, of the total cylindrical surface of roller 8.

The diameter of roller 8 is comprised between 300 and 800 mm and laminate 3 is heated at a temperature comprised between 50 and 60 °C when it is embossed by the embossing roller 8. For this purpose, the embossing roller 8 is pressed by thrust means, in particular pneumatic cylinders 10, against a cylinder 11 having an external surface 12 made of an elastomeric material, in particular a synthetic rubber containing chlorosulfonated polyethylene, for example Hypalon^{®}, having a hardness comprised between 60 and 70 Shore, in particular 65 Shore. Laminate 3 is preferably pressed between the embossing roller 8 and the elastomeric cylinder 11 so that nonwoven 6 is turned toward the embossing roller 8, while film 5 is turned toward the elastomeric cylinder 11.

Film 5 is made in particular with a polymeric compound mixed with fine mineral particles, in particular of CaC03, in a quantity comprised between 40% and 60% in weight with respect to the polymeric compound. This compound comprises in turn polyolefinic substances, in particular polyethylene and polypropylene. Film 5 is hot-laminated with nonwoven 6, in turn made up of polypropylenic fibers. The basis weight of film 5 is comprised between 20 g/m² and 25 g/m², in particular 22 g/m², while the basis weight of nonwoven 6 is comprised between 15 g/m² and 20 g/m², in particular 17 g/m².

With reference also to figure 4 and 5, it is seen that in a second embodiment of the invention, points 13 protruding from the cylindrical surface of the embossing roller 8 have substantially the same frustoconical shape of points 9 of the first embodiment, however their lateral surface is slightly concave outwards. Furthermore, pitch P between one point 13 and the four adjacent points is comprised between 1,9 and 2,2 mm, in particular 2,065 mm, while the area occupied by the upper surface of point 13 is a fraction comprised between 7% and 11%, in particular 9%, of the total cylindrical surface of roller 8.

In the process according to the present invention, the feeding speed of laminate 3 during the embossing in apparatus 1 is comprised between 0,5 and 3,5 m/s, while the pressure exerted by the embossing roller 8 on laminate 3 is comprised between 3 and 10 MPa.

Further variations and/or additions may be made by those skilled in the art to the embodiments hereinabove described and illustrated, while remaining within the scope of the following claims.

## Claims

1. Process for producing breathable laminates (3), **characterized in that** a laminate (3) comprising a polymeric film (5) laminated with a nonwoven (6) is embossed by means of at least one embossing roller (8) provided with a plurality of points (9; 13) protruding from its cylindrical surface.

2. Process according to the previous claim, **characterized in that** the embossing with the embossing roller (8) makes the laminate (3) breathable.

3. Process according to one of the previous claims, **characterized in that** the surface of the laminate (3) is embossed by the embossing roller (8) with a dot pattern.

4. Process according to one of the previous claims, **characterized in that** the points (9; 13) of the embossing roller (8) have a substantially frustoconical shape.

5. Process according to one of the previous claims, **characterized in that** the lamination and the embossing of the laminate (3) are carried out in a continuous manner one after the other.

6. Process according to one of the previous claims, **characterized in that** the polymeric film (5) includes fine mineral particles.

7. Process according to one of the previous claims, **characterized in that** the nonwoven (6) is turned toward the embossing roller (8) during the embossing of the laminate (3).

8. Process according to one of the previous claims, **characterized in that** the feeding speed of the laminate (3) during the embossing with the embossing roller (8) is comprised between 0,5 and 3,5 m/s.

9. Process according to one of the previous claims, **characterized in that** the pressure exerted by the embossing roller (8) on the laminate (3) is comprised between 3 and 10 MPa.

10. Process according to one of the previous claims, **characterized in that** the temperature of the laminate (3) during the embossing is comprised between 50 and 60 °C.

11. Apparatus (1) for producing breathable laminates (3), which comprises transport means (2) for transporting at least one laminate (3), **characterized in that** the apparatus comprises at least one embossing roller (8) provided with a plurality of points (9; 13) protruding from its cylindrical surface for embossing said laminate (3).

12. Apparatus (1) according to the previous claim, **characterized in that** the embossing roller (8) is provided with a plurality of points (9; 13) for embossing the surface of said laminate (3) with a dot matrix.

13. Apparatus (1) according to claim 11 or 12, **characterized in that** the points (9; 13) of the embossing roller (8) have a substantially frustoconical shape.

14. Apparatus (1) according to the previous claim, **characterized in that** the lateral surface of the points (13) is outwardly concave.

15. Apparatus (1) according to claim 13 or 14, **characterized in that** the diameter (D) of the upper surface of the points (9; 13) is comprised between 0,6 and 0,8 mm, in particular 0,7 mm.

16. Apparatus (1) according to one of claims 11 to 15, **characterized in that** the height (H) of the points (9; 13) is comprised between 0,7 and 0,9 mm, in particular 0,8 mm.

17. Apparatus (1) according to one of claims 11 to 16, **characterized in that** the points (9; 13) are arranged along a plurality of lines and columns.

18. Apparatus (1) according to the previous claim, **characterized in that** said lines and columns are inclined with respect to a generatrix (G) of the cylindrical surface of the embossing roller (8) with an angle (A) comprised between 40° and 50°, in particular 45°.

19. Apparatus (1) according to claim 17 or 18, **characterized in that** the pitch (P) between one point (9) and the adjacent points is comprised between 1,3 and 1,6 mm, in particular 1,46 mm.

20. Apparatus (1) according to claim 17 or 18, **characterized in that** the pitch (P) between one point (13) and the adjacent points is comprised between 1,9 and 2,2 mm, in particular 2,065 mm.

21. Apparatus (1) according to one of claims 11 to 20, **characterized in that** the area occupied by the upper surface of the points (9; 13) is a fraction comprised between 7% and 20%, in particular 9% or 18%, of the total cylindrical surface of the embossing roller (8).

22. Apparatus (1) according to one of claims 11 to 21, **characterized in that** the transport means (2) transport the laminate (3) during the embossing with a speed comprised between 0,5 and 3,5 m/s.

23. Apparatus (1) according to one of claims 11 to 22, **characterized in that** the embossing roller (8) presses the laminate (3) against a cylinder (11) having an external surface (12) made of an elastomeric material.

24. Apparatus (1) according to the previous claim, **characterized in that** the elastomeric surface (12) of said cylinder (11) is made with a synthetic rubber containing chlorosulfonated polyethylene.

25. Apparatus (1) according to claim 23 or 24, **characterized in that** the hardness of the elastomeric surface (12) of said cylinder (11) is comprised between 60 and 70 Shore, in particular 65 Shore.

26. Apparatus (1) according to one of claims 11 to 25, **characterized in that** the pressure exerted by the embossing roller (8) on the laminate (3) is comprised between 3 and 10 MPa.

27. Apparatus (1) according to one of claims 11 to 25, **characterized in that** the diameter of the embossing roller (8) is comprised between 300 and 800 mm.

28. Breathable laminate (3) comprising a polymeric film (5) laminated with a nonwoven (6), **characterized in that** the surface of the laminate (3) is embossed with a dot pattern.

29. Breathable laminate (3) according to the previous claim, **characterized in that** said dots are arranged along a plurality of lines and columns.

30. Breathable laminate (3) according to the previous claim, **characterized in that** said lines and columns are inclined with respect to a transversal axis with an angle (A) comprised between 40° and 50°, in particular 45°.

31. Breathable laminate (3) according to one of claims 28 to 30, **characterized in that** the polymeric film (5) includes fine mineral particles.

32. Breathable laminate (3) according to the previous claim, **characterized in that** said fine mineral particles are comprised in the film (5) in a quantity comprised between 40% and 60% in weight with respect to the polymeric compound.

33. Breathable laminate (3) according to the previous claim, **characterized in that** said polymeric compound comprises polyolefinic substances, in particular polyethylene and polypropylene.

34. Breathable laminate (3) according to one of claims 28 to 33, **characterized in that** the basis weight of the film (5) is comprised between 20 g/m² and 25 g/m², in particular 22 g/m².

35. Breathable laminate (3) according to one of claims 28 to 34, **characterized in that** the basis weight of the nonwoven (6) is comprised between 15 g/m² and 20 g/m², in particular 17 g/m².

## Amended claims

### Amended claims in accordance with Rule 86(2) EPC.

**1.** Process for producing breathable laminates (3), wherein a laminate (3) comprising a polymeric film (5) which includes fine mineral particles and is laminated with a nonwoven (6) is stretched for making the polymeric film (5) breathable by means of said particles, **characterized in that** said stretching is obtained by means of at least one embossing roller (8) provided with a plurality of points (9; 13) protruding from its cylindrical surface.

**2.** Process according to one of the previous claims, **characterized in that** the surface of the laminate (3) is embossed by the embossing roller (8) with a dot pattern.

**3.** Process according to one of the previous claims, **characterized in that** the points (9; 13) of the embossing roller (8) have a substantially frustoconical shape.

**4.** Process according to one of the previous claims, **characterized in that** the lamination and the embossing of the laminate (3) are carried out in a continuous manner one after the other.

**5.** Process according to one of the previous claims, **characterized in that** the nonwoven (6) is turned toward the embossing roller (8) during the embossing of the laminate (3).

**6.** Process according to one of the previous claims, **characterized in that** the feeding speed of the laminate (3) during the embossing with the embossing roller (8) is comprised between 0,5 and 3,5 m/s.

**7.** Process according to one of the previous claims, **characterized in that** the pressure exerted by the embossing roller (8) on the laminate (3) is comprised between 3 and 10 MPa.

**8.** Process according to one of the previous claims, **characterized in that** the temperature of the laminate (3) during the embossing is comprised between 50 and 60 °C.

**9.** Process according to one of the previous claims, **characterized in that** the lateral surface of the points (13) is outwardly concave.

**10.** Process according to one of the previous claims, **characterized in that** the diameter (D) of the upper surface of the points (9; 13) is comprised between 0,6 and 0,8 mm, in particular 0,7 mm.

**11.** Process according to one of the previous claims, **characterized in that** the height (H) of the points (9; 13) is comprised between 0,7 and 0,9 mm, in particular 0,8 mm.

**12.** Process according to one of the previous claims, **characterized in that** the points (9; 13) are arranged along a plurality of lines and columns.

**13.** Process according to the previous claim, **characterized in that** said lines and columns are inclined with respect to a generatrix (G) of the cylindrical surface of the embossing roller (8) with an angle (A) comprised between 40° and 50°, in particular 45°.

**14.** Process according to claim 12 or 13, **characterized in that** the pitch (P) between one point (9) and the adjacent points is comprised between 1,3 and 1,6 mm, in particular 1,46 mm.

**15.** Process according to claim 12 or 13, **characterized in that** the pitch (P) between one point (13) and the adjacent points is comprised between 1,9 and 2,2 mm, in particular 2,065 mm.

**16.** Process according to one of the previous claims, **characterized in that** the area occupied by the upper surface of the points (9; 13) is a fraction comprised between 7% and 20%, in particular 9% or 18%, of the total cylindrical surface of the embossing roller (8).

**17.** Process according to one of the previous claims, **characterized in that** the embossing roller (8) presses the laminate (3) against a cylinder (11) having an external surface (12) made of an elastomeric material.

**18.** Process according to the previous claim, **characterized in that** the elastomeric surface (12) of said cylinder (11) is made with a synthetic rubber containing chlorosulfonated polyethylene.

**19.** Process according to claim 17 or 18, **characterized in that** the hardness of the elastomeric surface (12) of said cylinder (11) is comprised between 60 and 70 Shore, in particular 65 Shore.

**20.** Process according to one of the previous claims, **characterized in that** the diameter of the embossing roller (8) is comprised between 300 and 800 mm.

**21.** Breathable laminate (3) comprising a microporous polymeric film (5) which is laminated with a nonwoven (6) and includes fine mineral particles, **characterized in that** the surface of the laminate (3) is embossed with a dot pattern.

**22.** Breathable laminate (3) according to the previous claim, **characterized in that** said dots are arranged along a plurality of lines and columns.

**23.** Breathable laminate (3) according to the previous claim, **characterized in that** said lines and columns are inclined with respect to a transversal axis with an angle (A) comprised between 40° and 50°, in particular 45°.

**24.** Breathable laminate (3) according to one of claims 21 to 23, **characterized in that** said fine mineral particles are comprised in the film (5) in a quantity comprised between 40% and 60% in weight with respect to the polymeric compound.

**25.** Breathable laminate (3) according to the previous claim, **characterized in that** said polymeric compound comprises polyolefinic substances, in particular polyethylene and polypropylene.

**26.** Breathable laminate (3) according to one of claims 21 to 25, **characterized in that** the basis weight of the film (5) is comprised between 20 g/m² and 25 g/m², in particular 22 g/m².

**27.** Breathable laminate (3) according to one of claims 21 to 26, **characterized in that** the basis weight of the nonwoven (6) is comprised between 15 g/m² and 20 g/m², in particular 17 g/m².
